# FASCICULE DE BREVET EUROPEEN

(11) **EP 0 574 283 B1**
(45) Date de publication et mention de la délivrance du brevet: **22.01.1997**
(21) Numéro de dépôt: 93401321.0
(22) Date de dépôt: 24.05.1993
(51) Int. Cl.: A01H 4/00

(54) **Procédé de tubérisation de pommes de terre**
Verfahren zur Knollenbildung von Kartoffeln
Method for the tuberization of potatoes

(30) Priorité: 26.05.1992 FR 9206425
(43) Date de publication de la demande: 15.12.1993
(73) Titulaire: COMMISSARIAT A L'ENERGIE ATOMIQUE, 75015 Paris Cédex 15 (FR)
(72) Inventeur: Chagvardieff, Pierre, F-84120 Pertuis (FR); Toussaint, Isabelle, F-13100 Aix en Provence (FR)
(74) Mandataire: Dubois-Chabert, Guy

(56) Documents cités:
- EP-A- 0 476 141
- WO-A-88/02213
- P.C. DEBERGH & R.H. ZIMMERMAN 'Micropropagation; technology and application', 1991 KLUWER ACADEMIC PUBLISHERS, DORDRECHT, pages 447-469; T.KOZAI:"Micropropagation under photoautotrophic conditions."
- AMERICAN POTATO JOURNAL vol. 59, 1982, pages 33-37, P-J. WANG & C-Y. HU 'In vitro mass tuberization and virus-free seed-potato production in Taiwan'
- NN 'Potato physiology, 1985, ACADEMIC PRESS, NEW YORK, Chapter 15 pages 503-377, P-J.WANG & C-Y. HU: "Potato tissue culture and its applications in agriculture"
- S.K. SEN & K.L. GILES - EDS- 'Plant cell culture in crop improvement' 1983, PLENUM PRESS, NEW YORK pages 9-18; L.E. HESZKY ET AL:"Tissue culture technology for long-term storage and propagation of potato (Solanum tuberosum L.) germplasms."
- ACTA HORTICULTURAE, vol. 230, 1988, pages 121-127; T. KOZAI et al 'Multiplication of potato plantlets in vitro with sugar free medium under high photosythetic photon flux'
- JOURN.JAPANESE SOCIETY HORTICULTUREAL SCIENCE, vol. 57, no. 2, 1988, pages 279-288, T. KOZAI & Y. IWANAMI 'Effects of CO2 enrichtment and sucrose concentration under high photon fluxes plantlet growth of carnation (Dianthus caryophyllus L.) in tissue culture during preparation stage'
- BLN 'Fast reproduction of potatoes by BLN, A potato programme for production of small tubers based on indexed, clean stock', 1986, BIOTEKNISK LABORATORIUM NIBE, NIBE

## Description

La présente invention concerne un procédé de tubérisation des stolons de pommes de terre, ce procédé permettant d'obtenir des mini tubercules. Les stolons sont des tiges souterraines.

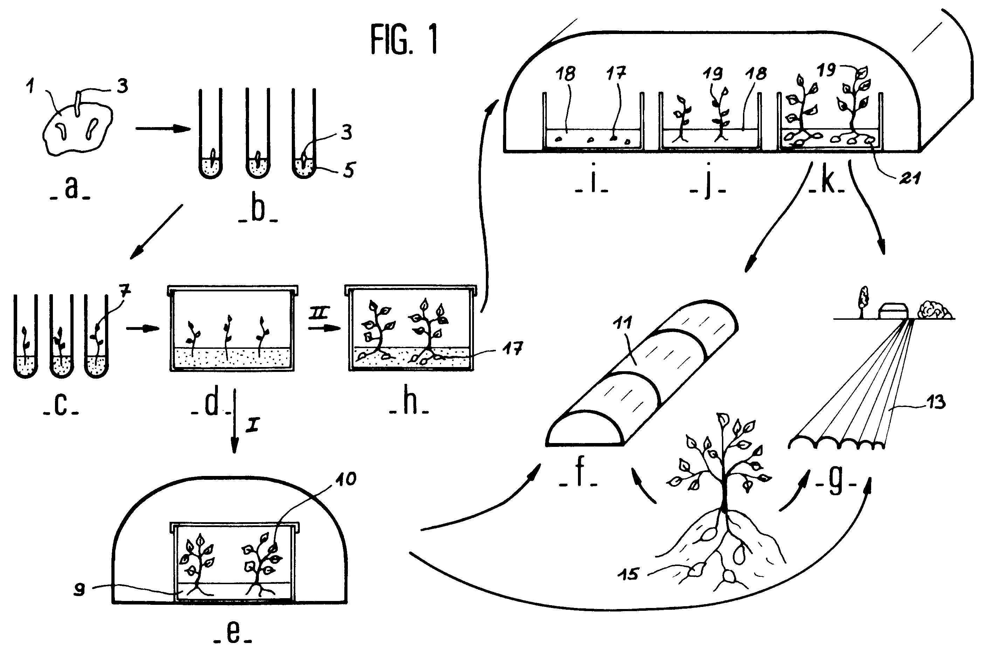

Un certain nombre de végétaux aptes à produire des tubercules, et notamment les pommes de terre, sont actuellement cultivés selon deux procédés différents illustrés sur les figures 1a à 1k jointes.

Comme illustré en figure 1a, on prélève sur un tubercule de pommes de terre 1, les bourgeons 3 qui se sont développés à sa surface. On les cultive in vitro (figure 1b), dans des tubes à essai par exemple, sur un milieu de culture 5 généralement minéral, contenant du sucre et parfois même des vitamines et/ou des hormones. Les bourgeons 3 se transforment en boutures 7 (figure 1c) et sont repiqués dans un bocal plus grand non ventilé en CO₂ (figure 1d) où ils se développent, toujours sur le milieu 5 hétérotrophe précité.

Selon un premier mode de réalisation (voie I), la bouture 7 obtenue est alors prélevée et replantée sur du terreau 9, et placée sous une serre (figure le). Au bout d'une certaine période de développement et d'acclimatation, les plants 10 obtenus peuvent être envoyés chez le producteur qui choisira de les cultiver sous serre 11 (figure 1f) ou dans les champs 13 (figure 1g), et qui obtiendra finalement après développement complet du plant de pommes de terre 10, des macrotubercules 15.

Un second mode de réalisation (voie II), consiste à réaliser l'étape de tubérisation des stolons in vitro, en milieu hétérotrophe, à partir des boutures 7 cultivées sur le milieu minéral 5. La tubérisation correspond à l'étape de transformation des stolons en tubercules. Cette étape (figure 1h) permet d'obtenir des microtubercules 17 d'une dimension inférieure à 10 mm. Ceux-ci seront ensuite replantés dans du terreau 18 (figure 1i) et cultivés sous serre, de façon à produire des jeunes plants 19 (figure 1j). Les stolons de ceux-ci se tubériseront alors pour donner des mini tubercules 21, d'une dimension comprise entre environ 10 et 28 mm (figure 1k). Ces mini tubercules 21 seront ensuite envoyés chez le producteur qui choisira de les cultiver sous serres (figure 1f) ou en pleine terre (figure 1g), afin d'obtenir les plants de pommes de terre et les macrotubercules 15 précédemment décrits.

Dans ce deuxième mode de réalisation, on constate que le temps s'écoulant entre l'étape a et l'étape h est d'environ 2 mois et que le temps s'écoulant entre l'étape i et l'étape k est d'environ 3 mois, soit un total de 5 mois pour obtenir des mini tubercules 21. Ceci est très long et il serait donc souhaitable pour des raisons de temps et de rentabilité financière que les producteurs de pommes de terre puissent obtenir ces mini tubercules dans un délai beaucoup plus court.

On connaît par ailleurs selon l'art antérieur, des procédés de culture in vitro, en conditions autotrophes, (c'est-à-dire sur un milieu exclusivement minéral, sans sucre ni vitamines), de plusieurs plantes. C'est ainsi que la culture d'oeillets a été réalisée sur milieu minéral seul, sous une atmosphère contenant 0,1 à 0,15% de CO₂ et dans des conditions d'éclairement telles que l'énergie lumineuse varie de 120 à 200 µE.m⁻².s⁻¹. Cette culture a été décrite dans un article de KOZAI et al. : J. Japan Soc. Hort. Sci. 57(2) 279-1988. De même, la culture des fraisiers sur milieu minéral dans une atmosphère contenant 0,2% de CO₂ et sous des conditions d'éclairement d'environ 200 µE.m⁻².s⁻¹ (énergie lumineuse) a été décrite dans un article de IWABUCHI et al. : "7st International Congress of Plant Tissue and Cell Culture", Amsterdam, 24-29 juin 1990.

Par ailleurs, la culture de pommes de terre a également été décrite dans deux articles de KOZAI et al., respectivement Acta Horticulturae 230. 121-1988 et "7st International Congress of Plant Tissue and Cell Culture", Amsterdam, 1990. Dans ces deux cas, les pommes de terre étaient cultivées sur milieu minéral, sous une énergie lumineuse voisine de 190 à 200 µE.m⁻².s⁻¹ et sous des teneurs en CO₂ voisines de 0,025 à 0,05%. Ces articles décrivent des procédés de culture en milieu autotrophe, mais dans le cas de la pomme de terre, aucune tubérisation n'a été observée.

La présente invention a donc pour but de réaliser un procédé de tubérisation, in vitro et dans des conditions autotrophes, des stolons de pommes de terre.

Selon les caractéristiques de l'invention, ce procédé de tubérisation comprend les étapes consistant à :
- repiquer des plants de pommes de terre dans une enceinte stérile contenant un milieu minéral autotrophe gélosé comprenant entre 3 et 5 mM d'ions NH₄⁺, entre 18 et 30 mM d'ions K⁺, entre 6 et 10 mM d'ions Mg²⁺, entre 12 et 20 mM d'ions Ca²⁺, entre 52 et 70 mM d'ions NO₃⁻, entre 3 et 5 mM d'ions PO₄³⁻ et entre 6 et 10 mM d'ions SO₄²⁻, cette enceinte étant maintenue sous une ventilation forcée d'air contenant entre 1 et 10% de CO₂, à température ambiante et sous des conditions d'éclairage correspondant à une photopériode comprise entre 16 et 18 heures par jour,
- maintenir ladite enceinte sous un éclairage faible, correspondant à une énergie lumineuse inférieure à 100 µE.m⁻²,s⁻¹, pendant une période comprise entre 8 et 12 jours, puis
- maintenir ladite enceinte sous un éclairage fort, correspondant à une énergie lumineuse comprise entre 300 et 500 µE.m⁻².s⁻¹, jusqu'à la tubérisation des stolons.

Grâce à ce procédé, on a réussi à obtenir, des mini tubercules de pommes de terre d'un diamètre compris entre 10 et 28 mm environ, à l'issue d'une période de 70 jours environ, au lieu de 5 mois habituels dans les procédés de l'art antérieur. Ce procédé permet donc d'obtenir une tubérisation deux fois plus rapide et beaucoup plus rentable puisque l'on obtient directement des mini tubercules. Au contraire, les tubercules obtenus sous des conditions hétérotrophes sont généralement beaucoup plus petits, (environ 10 mm) et nécessitent un passage en serre avant l'obtention de mini tubercules.

Par ailleurs, les mini tubercules obtenus par le procédé selon l'invention ont des caractéristiques spécifiques les différenciant des tubercules obtenus in vitro sous des conditions hétérotrophes ou en serres. A titre d'exemple, la teneur en protéine des tubercules de pommes de terre et l'activité de la PEP carboxylase (PhosphoEnolPyruvate carboxylase) ramenée à la masse sèche de tubercules, sont significativement plus élevées que les tubercules de pommes de terre de même taille obtenus en serres.

Enfin, ce procédé de tubérisation présente un intérêt du point de vue scientifique, car il permet d'étudier la tubérisation sous des conditions totalement contrôlées.

De façon avantageuse, les pommes de terre sont choisies parmi les variétés Haig, Kennebec, Denali, BF 15, Sirtema et Maris Page.

L'invention sera mieux comprise à la lecture de la description suivante d'un mode de réalisation préférentiel de l'invention, donné à titre d'exemple illustratif et non limitatif, cette description étant faite en faisant référence aux dessins joints, dans lesquels :
- les figures la à lk illustrent les diverses étapes d'un procédé de culture de pommes de terre de l'art antérieur, et

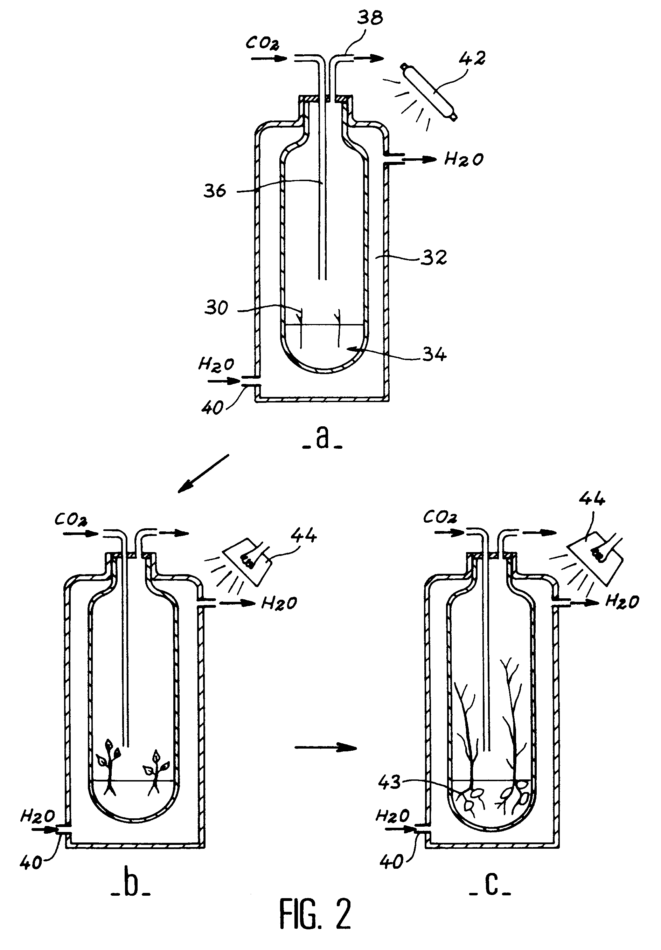

- les figures 2a à 2c illustrent les diverses étapes du procédé de tubérisation selon l'invention.

Comme illustré en figure 2a, le procédé de tubérisation selon l'invention consiste à repiquer des plants 30 de pommes de terre, à l'intérieur d'une enceinte stérile 32 de 1000 ml contenant environ 250 ml d'un milieu minéral autotrophe 34.

De façon avantageuse, mais non limitative, les plants 30 ont été obtenus à partir de boutures cultivées in vitro, sur un milieu minéral gélosé, enrichi en sucres et en vitamines.

L'intérieur de l'enceinte 32 est soumis à une ventilation forcée d'air enrichi en CO₂. Cette circulation s'effectue grâce à une canalisation d'entrée 36 et une canalisation de sortie 38. L'intérieur de l'enceinte est alimenté en eau en 40, afin de maintenir celle-ci à température ambiante, c'est-à-dire entre 20 et 30°C et de préférence à 25°C environ. En outre, l'enceinte est éclairée de façon à reproduire artificiellement les conditions d'éclairage du jour et de la nuit.

Les caractéristiques concernant chacun des paramètres qui viennent d'être évoqués vont maintenant être décrites plus en détail.

Des essais ont été effectués sur trois milieux de culture minéraux dont les quantités respectives en cations et en anions sont données dans le tableau 1 ci-dessous.

**Tableau 1**

| Elément | Teneur (mM) | | |
|---|---|---|---|
| Milieu | MS | 1H | 4H |
| NH₄⁺ | 20,6 | 1 | 4 |
| K⁺ | 20,0 | 6 | 24 |
| Mg²⁺ | 1,5 | 2 | 8 |
| Ca²⁺ | 3,0 | 4 | 16 |
| Na⁺ | 0,2 | 0 | 0 |
| NO₃⁻ | 39,4 | 14 | 56 |
| PO₄³⁻ | 1,25 | 1 | 4 |
| SO₄²⁻ | 1,7 | 2 | 8 |
| Cl⁻ | 6,0 | 0 | 0 |

Le premier milieu MS correspond au milieu connu sous la dénomination commerciale MURASHIGE et SKOOG, mis au point pour des conditions de culture hétérotrophe. Le deuxième milieu 1H correspond au milieu connu sous la dénomination commerciale HOAGLAND qui correspond aux besoins connus de la pomme de terre. Les essais ont également été effectués sur un milieu HOAGLAND concentré quatre fois et figurant dans le tableau sous la rubrique 4H. Les rapports entre les teneurs [PO₄³⁻]/[NO₃⁻+NH₄⁺] et [K⁺]/[NO₃⁻ + NH₄⁺] sont supérieurs dans les milieux 1H et 4H par rapport au milieu MS.

Des essais de tubérisation effectués in vitro, sur les trois milieux précités et sur la variété de pommes de terre Haig ont permis d'obtenir les résultats suivants, présentés dans le tableau 2.

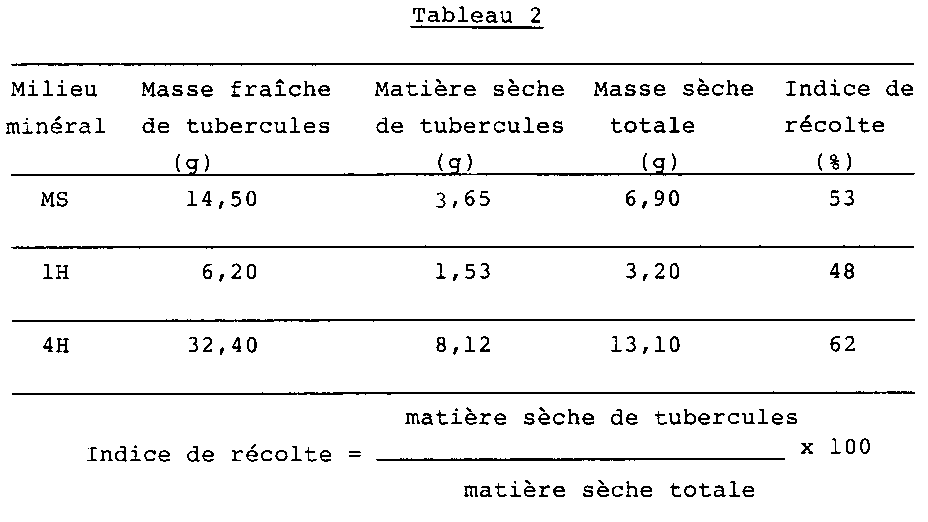

Les données de matière sèche de tubercules sont calculées à partir d'une teneur moyenne en matière sèche pour les tubercules. Ces comparaisons ont été effectuées après 70 jours de culture, sous une forte teneur en CO₂, une forte lumière et dans des bocaux de 1000 ml contenant 250 ml du milieu minéral gélosé testé. On peut donc constater que l'emploi du milieu minéral 4H permet d'obtenir une tubérisation présentant de bons indices de récolte. Des essais similaires non illustrés dans le tableau 2 ont également donné de bons résultats avec les variétés de pommes de terre Kennebec, Denali, BF 15, Sirtema et Maris Page.

On notera que si le milieu 4H a donné de bons résultats, il serait également possible d'utiliser comme milieu de culture autotrophe, un milieu 3H à 5H, c'est-à-dire dont la concentration des ions du milieu varie entre 3 et 5 fois celle du milieu de base HOAGLAND.

Par ailleurs, des essais complémentaires ont également été effectués, afin de déterminer la teneur en CO₂ et l'intensité lumineuse permettant d'obtenir la tubérisation la plus proche et la plus rapide possible. Des essais ont été effectués sur des plants de pommes de terre de variété HAIG cultivés dans des conditions autotrophes, c'est-à-dire dans une enceinte stérile contenant 125 ml de milieu minéral MS, sans sucre ni vitamines. On a fait varier l'énergie lumineuse et la teneur en CO₂. Les résultats obtenus sont illustrés dans le tableau 3 suivant.

**Tableau 3**

| Energie lumineuse (µE.m⁻².s⁻¹) | Teneur en CO₂ (%) | Masse fraîche de tubercules (g) | Durée de la culture (jour) |
|---|---|---|---|
| 120 | 0,03 | 6,0 | 136 |
| 120 | 2 | 1,6 | 70 |
| 450 | 0,03 | 0 | - |
| 450 | 2 | 7,0 | 70 |

Avant de passer aux valeurs d'énergie lumineuse indiquées dans la première colonne du tableau, on a d'abord laissé les plants pendant une dizaine de jours à environ 100 µE.m⁻².s⁻¹.

On peut constater que lorsque la teneur en CO₂ et l'intensité lumineuse sont faibles, on obtient une masse fraîche de tubercules intéressante (6 g), mais au bout d'une durée de tubérisation de 136 jours, c'est-à-dire une durée beaucoup trop longue pour que ce procédé de tubérisation présente un intérêt économique. Si l'on fait varier l'un des paramètres en augmentant, soit la teneur en CO₂, soit l'énergie lumineuse, on constate que la masse fraîche de tubercules obtenue est extrêmement faible. De façon totalement inattendue, en augmentant simultanément les deux paramètres (teneur en CO₂ et énergie lumineuse), on constate que l'on obtient une masse importante de tubercules, après une durée de tubérisation pratiquement deux fois plus courte (70 jours) que ce que l'on obtenait initialement dans les conditions de tubérisation précitées (1ère ligne du tableau).

En conséquence, il a été décidé de maintenir à l'intérieur de l'enceinte 32, une ventilation forcée d'air enrichi à 2% de CO₂. Toutefois, une teneur en CO₂ comprise entre 1 et 10% pourrait également convenir.

Il a également été décidé de maintenir l'enceinte pendant une dizaine de jours environ (figure 2a) sous des conditions d'éclairage faibles correspondant à une énergie lumineuse d'environ 100 µE.m⁻².s⁻¹, ou du moins ne dépassant pas cette valeur. Ces conditions sont obtenues par exemple, à l'aide d'un néon 42. Ces conditions sont nécessaires pour laisser les boutures s'acclimater après le repiquage jusqu'à formation des racines. Ensuite, pendant le début d'enracinement des plants 30 (figure 2b) et jusqu'à la tubérisation des stolons 43 (figure 2c), on maintient l'enceinte 32 sous un éclairage de forte énergie, comprise entre 300 et 500 µE.m⁻².s⁻¹ environ et de préférence 400 µE.m⁻².s⁻¹ environ. Ceci est obtenu par exemple à l'aide d'une ampoule 44 de forte intensité lumineuse.

Enfin, on a également constaté que contrairement à ce qui est recommandé en agronomie, il était possible de maintenir une température continue et assez élevée, comprise entre 20 et 30°C et plus précisément voisine de 25°C et une photopériode assez longue correspondant à 16 à 18 heures de lumière et 6 à 8 heures de nuit, par jour. Ces conditions ne sont pas inhibitrices de l'induction de la tubérisation et permettent, alliées à une forte teneur en CO₂ (2%) et à une forte lumière, d'augmenter la photosynthèse des plants.

Enfin, un dernier test comparatif a été effectué entre les tubercules de pommes de terre de variétés Haig et Kennebec obtenues en serres ou selon l'invention : sur milieu minéral autotrophe 4H, avec 2% de CO₂ et une forte lumière. Les tubercules ont été testés afin de déterminer d'une part, leur teneur en protéines, et d'autre part, l'activité de la PEP carboxylase, c'est-à-dire la PhosphoEnolPyruvate carboxylase. Les résultats sont illustrés dans le tableau 4 ci-après.

On constate que la teneur en protéines et l'activité de la PEP carboxylase sont plus élevées pour les tubercules obtenus par le procédé selon l'invention, que pour ceux obtenus en serre.

**Tableau 4**

| Variété | Mode d'obtention | Teneur en protéines mg/g MS | Activité PEPc | |
|---|---|---|---|---|
| | | | µM CO₂/h/ mg MS | µg CO₂/h/mg Prot. |
| HAIG | Serre Terreau-H/2 0,03 % CO₂ lumière du jour | 30,5 | 31,5 | 1,0 |
| | 4H-2 % CO₂ forte lumière | 48,3 | 59,2 | 1,2 |
| KENNEBEC | Serre Terreau-H/2 0,03 % CO₂ lumière du jour | 27,6 | 31,7 | 1,3 |
| | 4H-2 % CO₂ forte lumière | 51,2 | 57,0 | 1,2 |

## Revendications

1. Procédé de tubérisation des stolons de pommes de terre, caractérisé en ce qu'il comprend les étapes consistant à :
- repiquer des plants (30) de pommes de terre dans une enceinte stérile (32) contenant un milieu minéral autotrophe gélosé (34) comprenant entre 3 et 5 mM d'ions NH₄⁺, entre 18 et 30 mM d'ions K⁺, entre 6 et 10 mM d'ions Mg²⁺, entre 12 et 20 mM d'ions Ca²⁺, entre 52 et 70 mM d'ions NO₃⁻, entre 3 et 5 mM d'ions PO₄³⁻ et entre 6 et 10 mM d'ions SO₄²⁻, cette enceinte (32) étant maintenue sous une ventilation forcée d'air contenant entre 1 et 10% de CO₂, à température ambiante et sous des conditions d'éclairage correspondant à une photopériode comprise entre 16 et 18 heures par jour,
- maintenir ladite enceinte (32) sous un éclairage faible (42), correspondant à une énergie lumineuse inférieure à 100 µE.m⁻².s⁻¹ pendant une période comprise entre 8 et 12 jours, puis
- maintenir ladite enceinte (32) sous un éclairage fort (44), correspondant à une énergie lumineuse comprise entre 300 et 500 µE.m⁻².s⁻¹, jusqu'à la tubérisation des stolons.

2. Procédé de tubérisation selon la revendication 1, caractérisé en ce que l'air contient environ 2% de CO₂.

3. Procédé de tubérisation selon la revendication 1, caractérisé en ce que l'éclairage fort (44) correspondant à une énergie lumineuse voisine de 400 µE.m⁻².s⁻¹.

4. Procédé de tubérisation selon la revendication 1, caractérisé en ce que le milieu minéral (34) comprend environ 4 mM d'ions NH₄⁺, 24 mM d'ions K⁺, 8 mM d'ions Mg²⁺, 16 mM d'ions Ca²⁺, 56 mM d'ions NO₃⁻, 4 mM d'ions PO₄³⁻ et 8 mM d'ions SO₄²⁻.

5. Procédé de tubérisation selon la revendication 1, caractérisé en ce que les pommes de terre comprennent les variétés Haig, Kennebec, Denali, BF 15, Sirtema et Maris Page.

6. Procédé de tubérisation selon la revendication 1, caractérisé en ce que les plants (30) repiqués sont obtenus à partir de boutures cultivées, in vitro, sur un milieu minéral gélosé, enrichi en sucres et en vitamines.

## Claims

1. Method for the tuberization of potato stolons, characterized in that it comprises the steps consisting in:
- transplanting young potato plants (30) in a sterile chamber (32) containing an agar-based autotrophic inorganic medium (34) comprising between 3 and 5 mM NH₄⁺ ions, between 18 and 30 mM K⁺ ions, between 6 and 10 mM Mg²⁺ ions, between 12 and 20 mM Ca²⁺ ions, between 52 and 70 mM NO₃⁻ ions, between 3 and 5 mM PO₄³⁻ ions and between 6 and 10 mM SO₄²⁻ ions, this chamber (32) being maintained under a forced ventilation of air containing between 1 and 10% CO₂, at room temperature and under illumination conditions corresponding to a photoperiod of between 16 and 18 hours per day,
- maintaining the said chamber (32) under weak illumination (42) corresponding to a light energy below 100 µE.m⁻².s⁻¹ for a period of between 8 and 12 days, and then
- maintaining the said chamber (32) under strong illumination (44) corresponding to a light energy of between 300 and 500 µE.m⁻².s⁻¹, until tuberization of the stolons.

2. Method of tuberization according to Claim 1, characterized in that the air contains about 2% CO₂.

3. Method of tuberization according to Claim 1, characterized in that the strong illumination (44) corresponds to a light energy of close to 400 µE.m⁻².s⁻¹.

4. Method of tuberization according to Claim 1, characterized in that the inorganic medium (34) comprises about 4 mM NH₄⁺ ions, 24 mM K⁺ ions, 8 mM Mg²⁺ ions, 16 mM Ca²⁺ ions, 56 mM NO₃⁻ ions, 4 mM PO₄³⁻ ions and 8 mM SO₄²⁻ions.

5. Method of tuberization according to Claim 1, characterized in that the potatoes comprise the Haig, Kennebec, Denali, BF 15, Sirtema and Maris Page varieties.

6. Method of tuberization according to Claim 1, characterized in that the transplanted plants (30) are obtained from cuttings cultivated, in vitro, on an agar-based inorganic medium enriched with sugars and with vitamins.

## Patentansprüche

1. Verfahren zur Knollenbildung von Kartoffel-Stolonen, dadurch gekennzeichnet, daß es die Schritte umfaßt, die darin bestehen:
- Kartoffel-Pflänzlinge (30) in einen sterilen Behälter (32) umzusetzen, der ein autotrophes mineralisches Agar-Agar-Medium (34) enthält, das zwischen 3 und 5 mmol/l NH₄⁺-Ionen, zwischen 18 und 30 mmol/l K⁺-Ionen, zwischen 6 und 10 mmol/l Mg²⁺-Ionen, zwischen 12 und 20 mmol/l Ca²⁺-Ionen, zwischen 52 und 70 mmol/l NO₃⁻-Ionen, zwischen 3 und 5 mmol/l PO₄³⁻-Ionen und zwischen 6 und 10 mmol/l SO₄²⁻-Ionen enthält, wobei dieser Behälter (32) unter zwangsweiser Belüftung mit Luft, die zwischen 1 und 10% CO₂ enthält, auf Umgebungstemperatur und unter Beleuchtungsbedingungen, die einer täglichen Hellperiode zwischen 16 und 18 Stunden entsprechen, gehalten wird,
- den genannten Behälter (32) während eines Zeitraums zwischen 8 und 12 Tagen unter schwacher Beleuchtung (42) entsprechend einer Lichtenergie kleiner als 100 µE.m⁻².s⁻¹ zu halten, dann
- den genannten Behälter (32) bis zur Knollenbildung der Stolonen unter starker Beleuchtung (44) entsprechend einer Lichtenergie zwischen 300 und 500 µE.m⁻².s⁻¹ zu halten.

2. Verfahren zur Knollenbildung nach Anspruch 1, dadurch gekennzeichnet, daß die Luft ungefähr 2% CO₂ enthält.

3. Verfahren zur Knollenbildung nach Anspruch 1, dadurch gekennzeichnet, daß die starke Beleuchtung (44) einer Lichtenergie in der Nähe von 400 µE.m⁻².s⁻¹ entspricht.

4. Verfahren zur Knollenbildung nach Anspruch 1, dadurch gekennzeichnet, daß das mineralische Medium (34) ungefähr 4 mmol/l NH₄⁺-Ionen, 24 mmol/l K⁺-Ionen, 8 mmol/l Mg²⁺-Ionen, 16 mmol/l Ca²⁺-Ionen, 56 mmol/l NO₃⁻-Ionen, 4 mmol/l PO₄³⁻-Ionen und 8 mmol/l SO₄²⁻-Ionen enthält.

5. Verfahren zur Knollenbildung nach Anspruch 1, dadurch gekennzeichnet, daß die Kartoffeln die Sorten Haig, Kennebec, Denali, BF 15, Sirtema und Maris Page umfassen.

6. Verfahren zur Knollenbildung nach Anspruch 1, dadurch gekennzeichnet, daß die umgesetzten Pflänzlinge (30) aus Stecklingen erhalten werden, die in vitro auf einem mineralischen, mit Zuckern und Vitaminen angereicherten Agar-Agar-Medium kultiviert wurden.
